# EUROPEAN PATENT SPECIFICATION

(11) **EP 0 592 401 B1**
(45) Date of publication and mention of the grant of the patent: **23.08.1995**
(21) Application number: 91904290.3
(22) Date of filing: 13.02.1991
(51) Int. Cl.: A61F 13/46

(54) **AN ABSORBENT BODY INCORPORATING TWO LAYERS WHICH CONTAIN DIFFERENT SUPERABSORBENTS**
ABSORPTIONSGEGENSTAND MIT ZWEI UNTERSCHIEDLICHEN SUPERABSORBANZIEN ENTHALTENDEN LAGEN
CORPS ABSORBANT COMPOSE DE DEUX COUCHES QUI CONTIENNENT DIFFERENTS AGENTS SUPERABSORBANTS

(30) Priority: 14.02.1990 SE 9000534
(43) Date of publication of application: 20.04.1994
(73) Proprietor: Mölnlycke AB, 405 03 Göteborg (SE)
(72) Inventor: GUSTAFSSON, Lars, S-413 23 Göteborg (SE); ARESKOUG, Stefan, S-435 44 Mölnlycke (SE); QVIST, Magnus, S-448 00 Floda (SE)
(74) Representative: Kierkegaard, Lars-Olov
(86) International application number: SE9100101
(87) International publication number: WO9111978

(56) References cited:
- GB-A- 2 048 078
- SE-B- 463 747
- US-A- 4 673 402

## Description

The present invention relates to an absorbent body or pad for use in diapers, incontinence guards or like articles like those specified e.g. in GB-A-2 048 078.

It is highly essential that the absorbent pad of a diaper has a high absorption rate. The absorbent pad will normally exhibit a high absorption rate when dry, since the pulp core will then enclose a sufficient quantity of air which can be displaced rapidly and replaced with urine.

The fibres of the fluff mat in a dry absorbent pad are relatively rigid and are therewith able to withstand a certain amount of weight before becoming compressed. The fibres lose much of their ability to withstand weight or pressure when the pad is wet, therewith causing the fluff mat to collapse.

A large part of the pad volume is lost when the fluff mat has collapsed. The ability of the fluff mat to further absorb liquid is then restricted to the ability of the fluff to transport the urine from one location to another. The majority of fluffs manage this with differing degrees of success, although the process of transportation is very slow within the fluff mat and is constantly concerned with the transportation of excess urine. This excess is the difference between the amount of liquid present in the fluff at a particular moment and the ability of the fluff to retain liquid.

As mentioned, superabsorbents are able to absorb large quantities of urine, about 3-5 times the absorbency of fluff. Although this ability can be utilized, it is not particularly effective when solely one absorption ability is exchanged for the other. Superabsorbents can also contribute to other properties, such as improved surface dryness, etc., for instance.

The greatest obstacle to rapid, secondary absorption in a fluff mat which has collapsed at the first absorption, is that there is no air in the wet fluff to displace.

The present invention provides a solution to this problem.

The inventive absorbent pad is characterized in that the pad includes a first layer of fluff which, when the article is in use, lies nearest the wearer, a first super absorbent which is disposed in said fluff layer and which has a high degree of cross-linking and therewith the ability to swell while being substantially unaffected by normally occurring pressures, whereby subsequent to liquid absorption the collapsed pulp is loosened and again forms an air-containing voluminous fluff layer, and in that the absorbent pad includes a second layer in which there is disposed a second super-absorbent whose liquid absorbing capacity is greater than that of the first superabsorbent.

We have found that a superabsorbent having a very high degree of cross-linking, with subsequent high gel strength, is able to loosen the wet fluff while emptying the fluff of liquid at the same time. It will be understood that the total absorbency of the absorbent pad is not solely dependent on how much liquid the fluff and the superabsorbent can absorb individually.

The total absorbency is greatly dependent on the size of the volume that can be retained under the pressure forces that occur.

According to the invention, a highly cross-linked superabsorbent shall improve the rate at which a subsequent liquid discharge is absorbed, by loosening or "fluffing" the collapsed fluff mat and increasing the total volume of the wet region.

A superabsorbent which is so highly cross-linked that it can swell while being substantially unaffected by normally occurring pressures has a lower absorbency than a superabsorbent which has a lower degree of cross-linking. Consequently, the inventive absorbent pad includes a second layer which incorporates a second superabsorbent having a higher liquid absorbing capacity.

The invention will now be described in more detail with reference to the accompanying drawing, in which Figures 1 and 2 illustrate schematically the state of a cross-linked superabsorbent contained in a fluff mat, both before and after liquid absorption, whereas Figures 3 and 4 illustrate the corresponding states of a fluff mat containing a highly cross-linked superabsorbent.

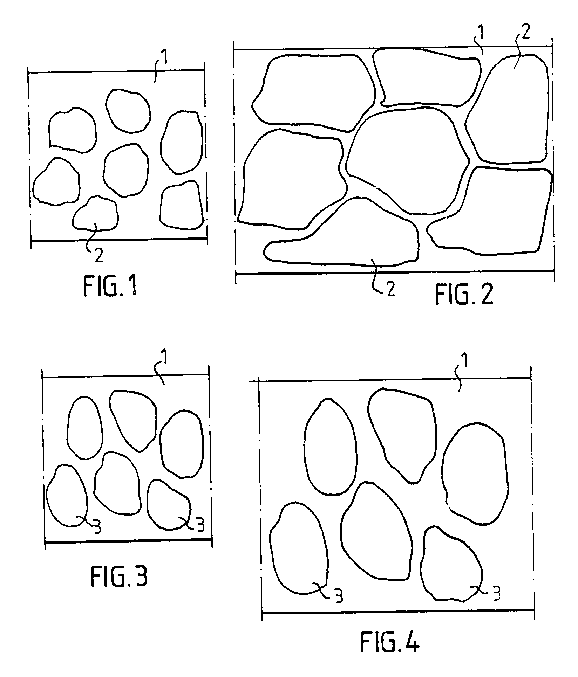

Figure 1 illustrates a fluff layer 1 in which grains or particles of cross-linked superabsorbent 2 are mixed. The fluff mat illustrated in Figure 1 is in a dry, voluminous state with an abundancy of air between the fibres. The absorbent pad shown in Figure 1 can therefore rapidly absorb liquid penetrating into the pad. When the fluff mat becomes wet, it collapses under the weight of absorbed liquid and under the pressure exerted by external loads and if no superabsorbents were present the collapsed fluff mat would discharge liquid when the absorbent pad is subjected to further, external pressures. The superabsorbents present in the fluff mat 1 take liquid from the fluff by suction and increase in size, by swelling. The superabsorbents 2 are cross-linked to an extent such as to possess sufficient gel strength to retain absorbed liquid under normal pressure in use, i.e. normal pressures on the absorbent pad of a diaper. As illustrated in Figure 2, the superabsorbents 2 do not form a continuous gel, but remain in the form of mutually separate grains or particles, even in their swollen state. As before-mentioned, the superabsorbents are also able to retain liquid when subjected to normal pressures in use, but do not retain their shape and are liable to be flattened laterally when subjected to pressure. The superabsorbents 2 are therefore not able to loosen the fluff, and the fluff mat will therefore remain substantially in a collapsed state. The absorbent pad shown in Figure 2 is therefore not able to quickly absorb further liquid.

Diaper manufacturers now desire superabsorbents whose gel strength will enable absorbed liquid to be retained effectively in the swollen gel even when subjected to pressure. The liquid absorbency of a superabsorbent decreases, however, with increased degrees of crosslinking and therewith increased gel strength.

We have found that there is at present no superabsorbent which has a sufficiently high liquid absorbency while being capable, at the same time, of loosening up a collapsed fluff mat.

Figure 3 illustrates a fluff mat 1 in which there are mixed superabsorbents 3 which have a high degree of cross-linking and therewith a high gel strength. As will be understood, the fluff mat 1 shown in Figure 3 will collapse in the same way as that described with reference to Figure 1 when the mat absorbs liquid. The superabsorbents 3, however, are so highly cross-linked and of such a high gel strength that they are able to swell under normal use pressure in a diaper or the like, without changing shape. Consequently, when the superabsorbent particles 3 swell, the fluff mat 1 will be loosened or "fluffed", as illustrated in Figure 4. The fluff mat is emptied of liquid, at the same time as it is loosened by the superabsorbent. Subsequent to swelling of the superabsorbent and loosening of the fluff mat, the absorbent pad illustrated in Figure 4 will contain a large amount of air which can be readily displaced when further liquid is absorbed.

The absorbent pad of the invention is constructed from an upper layer of the kind described with reference to Figures 3 and 4, this layer laying nearest the user when the absorbent pad is used in a diaper, and a bottom layer which includes a superabsorbent whose liquid absorbency is greater than the absorbency of the upper layer.

The bottom layer of the inventive absorbent pad may, for instance, be constructed in the manner described with reference to Figures 1 and 2.

The superabsorbent in the bottom layer may optionally comprise a superabsorbent having a gel strength which is so low as to form a continuous gel. The important criterion in this respect is that the upper layer is able to repeatedly absorb liquid quickly.

One example of a gel which exhibits a very high gel strength and which functions effectively in the upper layer of the inventive absorbent pad is Salsorb DPX 5038.

Good results have been achieved with Aqualic CA W-2 in an upper fluff layer and while using the similarly cross-linked superabsorbent Aqualic CA W-4 in the bottom layer. According to the manufacturer, Aqualic CA W-2 has a higher degree of cross-linking and therewith greater gel strength than Aqualic CA W-4.

All of the superabsorbents mentioned by way of example are cross-linked sodium polyacrylates.

It will understood that the invention is not limited to the aforedescribed embodiment and that several modifications can be made within the scope of the following claims.

For instance, an inventive absorbent pad may comprise an integrally configured fluff body which incorporates different layers of different superabsorbents.

## Claims

1. An absorbent body for use in diapers, incontinence guards or like articles, **characterized** in that the absorbent body includes a first layer of fluff (1) which lies nearest the wearer's body in use, a first superabsorbent (3) which is mixed in said layer and which has a high degree of cross-linking and therewith the ability to swell without being affected substantially by normally occurring pressure forces, whereby the fluff which collapses when absorbing liquid will be loosened and therewith again form an air-containing, voluminous fluff layer, and in that the absorbent body includes a second layer containing a second superabsorbent (2) having a higher liquid absorbency than the first superabsorbent.

2. An absorbent body according to Claim 1, **characterized** in that the second layer comprises fluff in which the second superabsorbent (2) is mixed.

3. An absorbent body according to Claim 1 or 2, **characterized** in that the second superabsorbent (2) in said second layer is cross-linked but has a lower gel strength than the first superabsorbent (1).

## Patentansprüche

1. Absorptionsgegenstand zur Verwendung in Windeln, in Kontinenzschutzvorrichtungen und ähnlichen Artikeln, dadurch **gekennzeichnet**, dass der Absorptionsgegenstand eine erste Flaumlage (1), die während der Verwendung dem Körper des Trägers am nächsten liegt, eine erste Superabsorbens (3), die der besagten Lage beigemischt ist und die einen hohen Vernetzungsgrad, und damit die Fähigkeit, von normalerweise auftretenden Druckkräften weitestgehend unbeeinflusst schwellen zu können, aufweist, wodurch der Flaum, der bei der Absorption von Flüssigkeit kollabiert, gelockert wird und damit wieder eine lufthaltige, voluminöse Flaumlage bildet, einschliesst, und dass der Absorptionsgegenstand eine zweite Lage einschliesst, die eine zweite Superabsorbens mit einer höheren Flüssigkeitsaufnahmefähigkeit als die erste Superabsorbens enthält.

2. Absorptionsgegenstand gemäss Anspruch 1, dadurch **gekennzeichnet**, dass die zweite Lage einen Flaum umfasst, dem die zweite Superabsorbens (2) beigemischt ist.

3. Absorptionsgegenstand gemäss Anspruch 1 oder 2, dadurch **gekennzeichnet**, dass die zweite Superabsorbens (2) in der zweiten Lage vernetzt ist, aber eine geringere Gelstärke als die erste Superabsorbens(1) aufweist.

## Revendications

1. Corps absorbant à utiliser dans des couches-culottes, des protections pour incontinents ou des articles similaires, caractérisé en ce que le corps absorbant contient une première couche de fluff (1) qui se trouve en cours d'utilisation le plus près du corps de l'utilisateur, un premier super-absorbant (3) qui est mélangé dans ladite couche et qui a un fort degré de réticulation et, donc, la capacité de gonfler sans être affecté de manière importante par des forces de pression se produisant normalement, ce qui fait que le fluff qui s'écrase quand il absorbe du liquide va être relâché et donc former de nouveau une couche de fluff volumineuse et contenant de l'air, et en ce que le corps absorbant comprend une seconde couche qui contient un second super-absorbant (2) ayant une plus forte capacité d'absorption de liquide que le premier super-absorbant.

2. Corps absorbant selon la revendication 1, caractérisé en ce que la seconde couche contient du fluff dans lequel est mélangé le second super-absorbant (2).

3. Corps absorbant selon la revendication 1 ou 2, caractérisé en ce le second super-absorbant (2) contenu dans ladite seconde couche est réticulé mais a une résistance de gel plus faible que le premier super-absorbant (1).
